# EUROPEAN PATENT SPECIFICATION

(11) **EP 4 085 256 B1**
(45) Date of publication and mention of the grant of the patent: **24.01.2024**
(21) Application number: 21702612.9
(22) Date of filing: 27.01.2021
(51) Int. Cl.: G01N 33/68

(54) **TEST AND IN VITRO DIAGNOSIS OF IRRITABLE BOWEL SYNDROME**
TEST UND IN-VITRO-DIAGNOSE VON REIZDARMSYNDROM
TEST ET DIAGNOSTIC IN VITRO DU SYNDROME DE L'INTESTIN IRRITABLE

(30) Priority: 27.01.2020 DE 102020101864
(43) Date of publication of application: 09.11.2022
(73) Proprietor: Immundiagnostik AG, 64625 Bensheim (DE)
(72) Inventor: ARMBRUSTER, Franz Paul, 64270 Bobenheim-Roxheim (DE); BASTIAN, Sonja, 64625 Bensheim (DE)
(74) Representative: Benedum, Ulrich Max
(86) International application number: PCT/EP2021/051858
(87) International publication number: WO 2021/151942

(56) References cited:
- WO-A1-2010/151699
- SIMREN MAGNUS ET AL: "INFLAMMATORY MARKERS IN THE FECES IN PATIENTS WITH THE IRRITABLE BOWEL", 20030401, vol. 124, no. 4, suppl. 1, 1 April 2003 (2003-04-01), XP009141791, DOI: 10.1016/S0016-5085(03)81983-2
- FUKUOKA ET AL: "Active monomers of human @b-tryptase have expanded substrate specificities", INTERNATIONAL HNMUNOPHARMACOLOGY, ELSEVIER, AMSTERDAM, NL, vol. 7, no. 14, 23 November 2007 (2007-11-23), pages 1900-1908, XP022360342, ISSN: 1567-5769, DOI: 10.1016/J.INTIMP.2007.07.007
- ROKA ET AL: "A Pilot Study of Fecal Serine-Protease Activity: A Pathophysiologic Factor in Diarrhea-Predominant Irritable Bowel Syndrome", CLINICAL GASTROENTEROLOGY AND HEPATOLOGY, ELSEVIER, AMSTERDAM, NL, vol. 5, no. 5, 2 May 2007 (2007-05-02), pages 550-555, XP022056341, ISSN: 1542-3565, DOI: 10.1016/J.CGH.2006.12.004

## Description

### FIELD OF THE INVENTION

The present invention relates to tests and methods for diagnosis of irritable bowel syndrome (ICD-10 K58), and to a biomarker for in vitro diagnosis of irritable bowel syndrome (IBS) in stool.

### BACKGROUND OF THE INVENTION

Irritable bowel syndrome (IBS) is a common gastroenterological condition which manifests itself by diffuse abdominal symptoms, bloating and flatulence, by recurrent abdominal discomfort, pains related to defecation, change in frequency, or in the consistency of stool or simply as spasmodic, dull-felt abdominal pain. Gastrointestinal motility, visceral hypersensitivity, cytokine release, alteration in fecal flora, and bacterial overgrowth, and psychosomatic illnesses have been associated with IBS. In most patients the bowel movement may also be altered uncomfortably in IBS to make them feel of constipation or diarrhea or a mixture of both.

Irritable bowel syndrome (IBS) is internationally classified by code ICD-10 K58, which includes subgroups based in the symptomatic manifestation: irritable bowel syndrome including irritable colon; irritable bowel syndrome including diarrhea predominant [IBS-D - K58.0]; irritable bowel syndrome including constipation predominant [IBS-C - K58.1]; irritable bowel syndrome including mixed bowel habits [IBS-M - K58.2]; unspecified irritable bowel syndrome (K58.8) and irritable bowel syndrome without diarrhea (K58.9). IBS-alternating (IBS-A) and post-infectious IBS (IBSPI) have also been described. The prevalence of IBS is between 10 and 20% with geographical variations: 21% in South America, 7% in Southeast Asia. The distribution among the subgroups IBS-D, IBS-C and IBS-M is largely the same in the USA, while in Europe IBS-D and IBS-C are the main groups, and IBS-M rather negligible. As a pathological cause, changes in intestinal motility, visceral sensitization, brain-intestinal interactions, shifts in the composition of the microbiome, changes in bile acid metabolism, and intestinal barrier disorders are discussed. More than 40% of IBS patients suffer so severely that their normal work routine, social interaction and sexual behavior are dramatically affected. The estimated health care cost of IBS in the United States is $8 billion per year (Talley et al., Medical costs in community subjects with irritable bowel syndrome, Gastroenterol. 1995, 109(6):1736-41).

According to ROM-IV criteria, irritable bowel syndrome (IBS) is present if the subject experienced abdominal pain or abdominal discomfort for at least 12 weeks including at least 2 of the following features: relief during defecation, change in stool frequency and consistency. Symptoms supporting the diagnosis are abnormal stool frequency (> 3/day or < 3/week) or abnormal stool consistency in more than 25% of the defecations, abnormal passage in more than 25% of defecations, mucus discharge in more than 25% of the defecations, flatulence or being exhausted longer than 25% of the time. The absence of any structural or biochemical disorders that could be causing the symptoms is also a necessary condition. As a result, the Rome IV criteria can only be considered in case there is a reliable patient history with absence of abnormal intestinal anatomy or metabolic process which would otherwise explain the symptoms (Lacy BE et al. in Bowel Disorders Gastroenterology 2016;150:1393-1407)

The diagnosis of IBS is up to now an exclusion of other diseases such as colorectal carcinoma, ovarian cancer, chronic inflammatory diseases, microscopic colitis, and celiac disease. Additionally, other diseases like malabsorption of lactose or fructose, bacterial overgrowth syndrome (SIBO), bile acid malabsorption, non-celiac wheat sensitivity (NCGS), diverticulitis, intestinal ischemia, disorders of intestinal motility and gynecological disorders. Examination includes a detailed anamnesis, abdominal and rectal examination, blood- and stool-testing for the above-mentioned diseases (intestinal inflammation markers, celiac disease markers and pathogens), sonography of the abdomen and gynecological examination. The diagnosis approach includes besides anamnesis, abdominal palpation, and sonography a performing of complete blood counts (CBC), biochemical profiles (including liver tests), serologic markers for celiac disease as well as stool examination for ova and parasites in patients with diarrhea predominance, determination of thyroid-stimulating hormones and calcium for patients with constipation, and flexible sigmoidoscopy, endoscopy and colonoscopy.

The etiology of irritable bowel syndrome is unclear but appears to involve both psychosocial and physiologic factors (stress, anxiety, depression, trauma, and abuse). Numerous other diseases may therefore be confused with IBS, including food hypersensitivity, lactose intolerance, drug-induced diarrhea, diarrhea related to post-cholecystectomy, laxative abuse, parasitic diseases, eosinophilic gastritis or enteritis, microscopic colitis, small-bowel bacterial overgrowth, celiac disease, and early inflammatory bowel disease. Older age, fever, weight loss, rectal bleeding or vomiting are also parameters excluding a diagnosis of IBS. A diagnosis of IBS can only be considered if, despite careful examination of the patient, no organic causes for existing abdominal complaints can be found.

IBS diagnosis and assessment of its severity is therefore complex as there are no specific biological, radiographic, or physiological markers which can be used for a positive diagnosis or its clinical cause. The incomplete understanding of its pathobiology and the large number of symptoms have allowed no definition of a reliable biomarker or list of positive symptoms for IBS. Diagnostic methods for positively distinguishing IBS from other gastrointestinal disorders displaying similar symptoms, notably psychosomatic conditions and hypochondriasis, are currently not available. The state of the art therefore represents a problem.

### SUMMARY OF THE INVENTION

A solution to the stated problem is provided by a method of diagnosis of irritable bowel syndrome (IBS) and its severity in a patient suffering from gastrointestinal disorders, comprising the steps of:-
preparing an defined extract of fecal proteins from a stool sample of a patient suffering from gastrointestinal disorders in a buffer system which allows a stable definition of a reference range;
determining the presence and concentration of intestinal tryptase (γ-tryptase) in said fecal protein extract using immunological methods;
comparing said measured concentration of intestinal tryptase in said fecal protein extract to a predetermined reference value; and
assessment of said patient by assigning an increased likelihood of irritable bowel syndrome (IBS) when said measured concentration of intestinal tryptase (γ-tryptase) in said fecal protein extract is higher than a predetermined reference value, or by assigning a decreased likelihood of irritable bowel syndrome (IBS) when said measured concentration of intestinal tryptase (γ-tryptase) in said fecal protein extract is lower than said predetermined reference value.

A preferred embodiment of the method comprises the use of antibodies raised against human γ-tryptase isolated from lung tissue, and more preferred the use of monoclonal antibodies specifically binding human γ-tryptase.

In some embodiments, the immunological methods comprise the steps of i) providing an antibody raised against total mast cell tryptase which antibody is bound to a solid phase; ii) contacting said antibody against total mast cell tryptase with said fecal protein extract and providing conditions for the formation of a first immune complex; iii) providing a second antibody specific for human γ-tryptase and providing conditions to allow formation of a sandwich immune complex; and iv) determining the amount of sandwich immune complex formed to determine the amount of γ-tryptase in said fecal protein extract. The immunological method may be an enzyme-linked immunosorbent assay (ELISA). In some embodiments, the method may be an immunoturbidimetric or immunonephelometric assay.

The predetermined reference value may be determined in stool from healthy subjects and said reference value may be 10 ng γ-tryptase per gram feces. Another predetermined reference value may be 20 ng γ-tryptase per gram feces, which reference value is associated with an increased likelihood of irritable bowel syndrome subtypes IBS-Constipation / IBS-Mixed. A further predetermined reference value is 30 ng γ-tryptase / g feces which is associated with an increased likelihood of a diagnosis of irritable bowel syndrome subtype IBS-Diarrhea. If not the intestinal tryptase (γ-tryptase) is determined in feces but total tryptase including α-- and β-tryptase from activated mast cells in blood and serum, then the total tryptase concentrations in feces are higher and no longer specific for diagnosis of irritable bowel disease.

In some embodiments, the fecal protein extract may be further examined for the presence of one or more of the following fecal biomarkers: calprotectin, pancreatic elastase, lactoferrin, hemoglobin, hemoglobin-haptoglobin-complex, anti-transglutaminase antibodies, anti-gliadin-antibodies, secretory IgA, α-1-trypsin, albumin, EDN, lysozyme, β-defensin, bile acids. This may be done simultaneously or together with the examination for intestinal tryptase for a positive diagnosis of the numerous non-IBS diseases. The diagnosis of the gastrointestinal disorder may comprise an anamnesis or assessment and evaluation of at least one criteria or symptom selected from older age, fever, weight loss, rectal bleeding, vomiting, lactose intolerance, drug-induced diarrhea, postcholecystectomy syndrome, laxative abuse, parasitic diseases, eosinophilic gastritis or enteritis, microscopic colitis, small-bowel bacterial overgrowth, celiac disease, early inflammatory bowel disease, abdominal pain, abdominal discomfort, constipation, diarrhea, bloating and/or abdominal distension,

Another aspect of the present disclosure is related to a kit of parts for determining the type and severity of a gastrointestinal disorder in a subject suspected of suffering from irritable bowel syndrome or hypochondriasis, comprising a) a device for transferring a defined amount of feces in a buffer system to prepare an extract of fecal proteins; and b) reagents for determining the concentration of γ-tryptase in said extract of fecal proteins.

The kit of the invention is defined in the appended claims. In some embodiments the kit comprises a) a reagent containing antibodies capable of binding total mast cell tryptase and forming a first complex; and b) a reagent containing antibodies raised against γ-tryptase isolated human lung tissue and specifically binding γ-tryptase; and c) reagents for a quantitation of the antibody-γ-tryptase complex formed.

A preferred embodiment of the invention relates to a kit comprising a) a reagent containing antibodies capable of binding total mast cell tryptase and forming a first complex; and b) a reagent containing monoclonal antibodies specifically binding γ-tryptase; and c) reagents for a quantitation of antibody-γ-tryptase complex formed.

The present disclosure also relates to a test and method of monitoring irritable bowel syndrome (IBS) and its severity in a patient, comprising the steps of obtaining a defined fecal sample and extracting said sample in a buffer solution to obtain an extract of solubilized fecal proteins; determining the presence and concentration of γ-tryptase in said fecal sample extract; comparing said measured concentration of γ-tryptase with previous measurements. The method may comprise an assessment of said patient suffering from irritable bowel syndrome (IBS) when said measured concentration of γ-tryptase is higher or lower than a predetermined value. The test and method may include a determination of calprotectin and/or lactoferrin in said very same fecal sample extract.

The test and method may be a homogenous immunoassay or an enzyme-linked sandwich immunoassay using immobilized capture anti-tryptase antibodies and specific antibodies against γ-tryptase.

The test and method allow not only a positive diagnosis but also a monitoring of the severity of IBS and potential other gastrointestinal disorders. The disclosure provides clinicians with a biomarker an assessment of the severity of the irritable bowel syndrome since the amount of fecal γ-tryptase will correlate with the intensity of the pains and discomfort of the patient and, most importantly, the instant disclosure provides a physiological biomarker which allows the clinician or general practitioner a differentiation of patients suffering from IBS and subjects suffering from hypochondriasis as a purely mental disorder.

In one embodiment, the method of determining the amount or concentration of γ-tryptase in a stool sample may comprise a provision of immobilized antibodies raised against human α-/β-tryptase contained in the secretory granules of mast cells; contacting said antibodies with said stool sample extract and providing conditions for the formation of a first immune complex; providing antibodies raised against human γ-tryptase isolated from lung tissue, which antibodies are capable of specifically binding to epitopes specific for transmembrane γ-tryptase, and contacting said anti-γ-tryptase-antibodies with said first immune complex and providing conditions for the formation of an immune sandwich complex, and determining the concentration of formed sandwich complex. The epitope specific for fecal γ-tryptase may be three-dimensional epitope.

In a preferred embodiment, said predetermined reference value may be 10 ng γ-tryptase per gram fecal sample. In a further embodiment the method may comprise a comparing of the measured concentration of γ-tryptase to a second predetermined reference value, when the patient is suffering from irritable bowel syndrome subtypes IBS-C (constipation) or IBS-M (mixed). In some embodiments, the method may comprise a comparing of the measured concentration of γ-tryptase to a reference value of 30 ng γ-tryptase / g fecal sample, when the patient is suffering from irritable bowel syndrome subtype IBS-D (diarrhea). In this case, it may be advisable to determine the concentration of bile acids in said fecal sample extract, too. Thus, the disclosure provides a fecal biomarker supporting a classification of a diagnosed IBS in accordance with IBS-constipation (IBS-C), IBS-diarrhea (IBS-D), IBS-mixed (IBS-M), IBS-alternating (IBS-A), or post-infectious IBS (IBSPI).

Another aspect of this patent application relates to a kit of parts for monitoring the type and severity of a gastrointestinal disorder in a subject which reagents comprise antibodies binding to mast cell specific tryptase and antibodies specific for γ-tryptase as well as reagents for determining the amount of complex formed. In some embodiment the kit may comprise a solid phase with immobilized antibodies binding total tryptase and second antibodies raised against transmembrane tryptase from human lung tissue, which second antibodies are specific for γ-tryptase, and reagents for detection and quantitation of said sandwich complex.

Further aspects and advantages of embodiments of the present invention will become apparent from the following detailed description, taken in conjunction with the accompanying drawings, illustrated by way of example of the principles of the invention.

### BRIEF DESCRIPTION OF THE DRAWINGS

In the figures and drawings appended hereto:
- **Fig. 1**: is a diagram comparing γ-tryptase concentrations in stool of healthy subjects and patients suffering from irritable bowel syndrome IBS-C, IBS-M, IBS-D and allergic reactions.
- **Fig. 2**: is a diagram comparing intestinal total tryptase concentrations in stool of healthy individuals (C), individuals with a high risk for IBS (asthma, psoriasis, food allergy, rheumatoid arthritis) and individuals with IBS in therapy diagnosed in accordance with ROME IV;

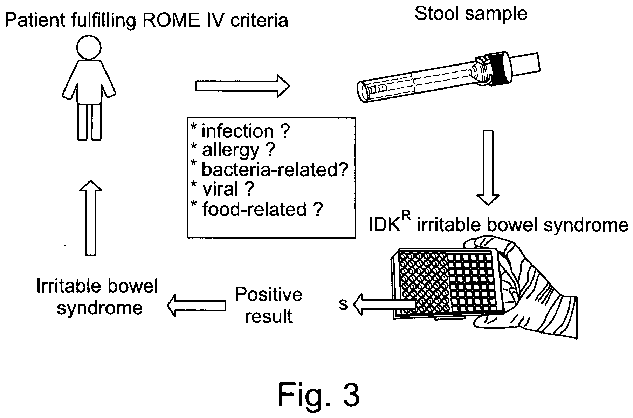

- **Fig. 3**: is a schematic representation of the steps to diagnosis of irritable bowel syndrome in accordance with ROME IV.

### DETAILED DESCRIPTION OF THE INVENTION

The invention is defined in the appended claims.

The present application for a grant of patent provides a positive approach to an *in vitro* diagnosis of irritable bowel syndrome (IBS) in a patient displaying diffuse gastrointestinal disorders. Although medical guidelines for the management of irritable bowel syndrome (IBS) state that invasive tests, such as colonoscopy, are not required for diagnosis, clinicians and general practitioners generally undertake invasive tests to exclude potential organic causes. Their reluctance to a positive approach to diagnosing IBS are due to concerns of a missed organic pathology, notably colorectal cancer. In addition, the symptom-based criteria for diagnosis of IBS are unwieldy and perform only modestly having regard that the etiology of IBS is multifactorial and of composite nature. Particular attention must be given to the character of the pain, bowel habits, familial interrelationships, and drug and dietary histories as well as to the patient's overall emotional state, interpretation of personal problems, and quality of life.

The pathophysiology of IBS is not well understood. There are reports suggesting a dysregulation of the gastrointestinal microbiota in the gut. Other studies report that stress hormones as well as cortisol levels are elevated in IBS patients (Heitkemper et al., Increased urine catecholamines and cortisol in women with irritable bowel syndrome, Am J Gastroenterol, 1996; 91(5):906-13). In some IBS patients an increased number of mast cells was found in the gastrointestinal mucosa (Guilarte, M. et al., Diarrhoea-predominant IBS patients show mast cell activation and hyperplasia in the jejunum, Gut 2007; 56, 203-209). Other studies report increased levels of mediators, including histamine and serine proteases in the colonic mucosa (Buhner et al., Activation of human enteric neurons by supernatants of colonic biopsy specimens from patients with irritable bowel syndrome, Gastroenterology, 2009; 137(4)). The serotonin pathway has further been implicated in the regulation of gastrointestinal motility, secretion, and sensation as well as imbalances affecting the enteric nervous system. Alterations of the tryptophan/serotonin/kynurenine metabolic and catabolic pathways have been described as cause for IBS-D (Christmas et al., Increased serum free tryptophan in patients with diarrhea-predominant irritable bowel syndrome, Nutrition Research, 2010, 30:678-688). Some studies suggest a heightened sensitivity to visceral pain perception, also known as peripheral sensitization, which may be due to a reduction in the threshold of activation of the transduction processes of primary afferent neurons. A variety of mediators including monoamines (e.g., catecholamines and indoleamines), cytokines and prostanoids, such as E-type prostaglandins may have a role in that (Mayer et al., Basic and clinical aspects of visceral hyperalgesia, Gastroenterol., 1994; 107:271-293). Intestinal motor dysfunction has been described as causative for IBS and abnormal handling of intraluminal contents and/or gas formation (Kellow et al., Altered small bowel motility in irritable bowel syndrome is correlated with symptoms, Gastroenterol. 1987; 92:1885-1893). This is because the walls of the intestines are in line with layers of muscle which contract and relax as food is transported from the stomach through the intestinal tract down to the rectum. These contractions are said to be stronger and longer lasting in IBS patients than in healthy subjects so that food is locally moved faster causing gas, bloating, and diarrhea. In other cases, the opposite occurs: a slowed food passage let stool become dried and harder, causing constipation. While gut dysmotility and altered visceral perception are important contributors to symptom pathogenesis, this condition is now generally viewed as a disorder of the brain-gut axis (Mayer et al., Basic and clinical aspects of visceral hyperalgesia, Gastroenterol., 1994; 107:271-293). As mentioned, psychological factors in conjunction with disorders such as depression and anxiety may also lead to IBS (Drossman et al., Sexual and physical abuse and gastrointestinal illness, Scand J Gastroenterol Suppl. 1995; 208:90-6) and there are patients with hypochondriasis.

Since patients with IBS can develop additional gastrointestinal disorders and diseases, testing for celiac disease, IBD, colorectal cancer and other pertinent diseases must always be considered. Changes in symptoms (e.g., in the location, type, or intensity of pain; in bowel habits; in constipation and diarrhea) and new symptoms or complaints (e.g. nocturnal diarrhea) may signal another disease process and therefore a monitoring of fecal γ-tryptase is advisable in IBS patients. Other symptoms that require investigation include blood in the stool, weight loss, severe abdominal pain or unusual abdominal distention, steatorrhea or noticeably foul-smelling stools, fever or chills, persistent vomiting, hematemesis, symptoms disturbing sleep (e.g., pain, the urge to defecate), and a steady progressive worsening of symptoms. IBS like symptoms may also be due to drug-induced diarrhea, laxative abuse, parasitic diseases (e.g., giardiasis).

A factor which contributes further to the difficulty in the diagnosis of IBS is the bimodal (younger and older) age distribution of patients with gastrointestinal disorders. Patients +40 years of age are more likely to develop an intercurrent physiologic illness. Patients +50 years of age should receive colonoscopy to exclude colonic polyps and tumors. In patients +60 years of age ischemic colitis must be considered. Patients with constipation and no anatomic lesion should be examined for hypothyroidism and hypercalcemia. Rare causes of diarrhea include hyperthyroidism, medullary cancer of the thyroid, or carcinoid syndrome, gastrinoma, and vipoma. However, these causes of diarrhea are typically accompanied by stool volumes >1000 mL daily, which differentiates them from IBS. If the symptoms suggest malabsorption, tropical sprue or celiac disease must be considered.

Many patients with IBS are over tested: by analyzing the subject's complete blood count, biochemical profile (including liver tests), serologic markers for celiac disease (tissue-transglutaminase IgA with an IgA level), stool examination for ova and parasites (in patients with diarrhea predominance), thyroid-stimulating hormone and calcium for patients with constipation, and flexible sigmoidoscopy or colonoscopy. During flexible fiberoptic proctosigmoidoscopy, introduction of the instrument and air insufflation frequently triggering bowel spasm and pain, the mucosal and vascular patterns usually appear normal in IBS. In patients with chronic diarrhea, particularly older women, mucosal biopsy can rule out possible microscopic colitis. Additional studies (such as ultrasonography, CT, barium enema x-ray, upper GI esophagogastroduodenoscopy, and small-bowel x-rays) must usually be undertaken when there are other objective abnormalities. Fecal fat excretion should be measured when there is a concern about steatorrhea. Small-bowel evaluation (e.g., enteroscopy, capsule endoscopy) is recommended when malabsorption is suspected. Testing for carbohydrate intolerance or small-bowel bacterial overgrowth should be considered in appropriate circumstances.

Up to 80% of IBS patients subjectively identify, without any testing, "food" as a potential trigger of their symptoms. Food allergy has been classically related to IBS by means of a "mucosal prick test" known as Colonoscopic Allergen Challenge (COLAP). 77% of the irritable bowel patients examined by the COLAP test showed a positive result. These findings indicate that allergic or other immunological reactions to food may be involved in the development of symptoms of irritable bowel syndrome (Borghini et al. in New insights in IBS-like disorders: Pandora's Box has been opened; a review. Gastroenterology and Hepatology From Bed to Bench 2017, 10(2):79-89). Alternatively, a measurement of total tryptase in feces or serum may be used to determine whether the symptoms result from activated mast cells.

A diagnosis of irritable bowel syndrome is therefore extremely challenging in view of the symptomatic similarity between IBS and other gastrointestinal disorders. Biomarker-based assays can therefore provide objective criteria for distinguishing IBS from other disorders. To our knowledge, we report for the first time that the level of fecal γ-tryptase can serve as a biomarker for an assessment and monitoring of irritable bowel syndrome (IBS). The present description provides a method and test for diagnosing irritable bowel syndrome (IBS) in a subject. The methods comprise measuring the concentration of γ-tryptase in an extract of a fecal sample and that an increased likelihood of IBS can be inferred from the measured level of γ-tryptase in comparison to a healthy control. The present disclosure provides a fecal biomarker for diagnosis of IBS and/or IBS subtypes and an objective marker for differentiation of patients with IBS from subjects suffering from hypochondriasis.

The general practitioner consulting with a patient with diffuse gastrointestinal disorders in primary care can exclude numerous organic gastrointestinal (GI) diseases by testing a fecal sample from said patient for calprotectin and lactoferrin. The corresponding immunoassays have been extensively validated. These biomarkers have a high negative predictive value for an absence of an inflammation (IBD) or endoscopic lesions. There is less of a correlation between plasma CRP levels and clinical indices. Analyzing a stool or plasma sample is much less invasive than performing a colonoscopy and being able to eliminate the need for colonoscopy is important, especially with pediatric patients. A testing of fecal biomarkers is further psychologically advantageous because seemingly directed to the bowel and patient's complaints. Further predictive biomarkers for organic GI diseases and/or reflecting gut integrity are hemoglobin (occult blood), hemoglobin-haptoglobin, stool oncostatin M, secretory IgA, stool anti-gliadin-slgA, stool anti-htTG-slgA, plasma zonulin (Immundiagnostik AG, Bensheim, DE, article no. K5600), plasma C-reactive protein (CRP).

Calprotectin and lactoferrin are proteins of neutrophil granulocytes in the mucosa and whenever there is an inflammation, they will become released and detectable in feces because highly resistant to an enzymatic breakdown. The present application however discloses a biomarker for irritable bowel syndrome (IBS) in the absence of an inflammation. A determination of γ-tryptase in a fecal sample extract therefore provides a positive approach to the *in vitro* diagnosis of IBS. The complementary testing for fecal γ-tryptase in case of diffuse GI disorders still requires a testing for calprotectin and lactoferrin but calprotectin, lactoferrin, occult blood and γ-tryptase can be determined in one fecal sample extract of said patient.

As used herein, the term "total tryptase" stands for the total α-/β-tryptase (tryptase-1 - EC:3.4.21.59) of mast cells. The human tryptases were initially divided into two groups, α (alpha) and β (beta). α-Tryptase is the major circulating isoform and expressed by basophils while β-tryptases is the major type stored in secretory granules of neutrophil granulocytes. Structurally, human α/β-tryptases are closely related. β-Tryptases are 98-99% identical in amino acid sequence. α-Tryptases are less closely related (α1 is 91% identical with β1). The mast cells control by unknown mechanisms the paracellular permeability of the intestinal epithelium during parasitic infection, allergic inflammation and/or chronic stress (Jacob C et al. in Mast Cell Tryptase Controls Paracellular Permeability of the Intestine, J. Biol. Chem. 280: 31936-31948 (2005). The secretory granules further contain histamine, proteases, and cell signaling proteins such as TNF-α which are released into the lumen upon a coupled activation-degranulation response, e.g. in the acute phase reaction, stress-associated conditions, allergic reactions as well as in systemic inflammations. The pathologic roles of α/β-tryptases in diseases where mast cell activation is suspected such as asthma have not been deduced. Notwithstanding, the human α/β-tryptases levels in serum are therefore proven clinical tools for the evaluation of systemic anaphylaxis and systemic mastocytosis. Even though the primary amino acid sequences of α- and β- tryptase are 93% identical, it should be noted that these two mast cell proteases are functionally distinct due, in part, to a single amino acid difference in one of the loops that forms the substrate binding cleft of each tryptase.

The term "γ-tryptase" (or *"intestinal tryptase"*) stands for protein of the γ-tryptase gene, *TPSG1,* which encodes a type I transmembrane serine peptidase that is C-terminally anchored to the cell surface after secretion (Wong GW et al in Identification of a new member of the tryptase family of mouse and human mast cell proteases which possesses a novel COOH-terminal hydrophobic extension, J. Biol. Chem. 1999, 274:30784-30793). The intron-exon organization of the tryptase genes is unique, more closely related to the protease prostasin, and suggests that the tryptases belong to the serine peptidase family (Caughey et al. Characterization of Human γ-tryptases, Novel Members of the Chromosome 16p Mast Cell Tryptase and Prostasin Gene Families, J Immunol 2000, 164:6566-6575). The membrane-spanning domain of γ-tryptase is analogous to tryptic-like convertase furine which suggests that γ-tryptase is taking part in the post-translational processing of proteins in the secretory granules. γ-tryptase can become inactivated by human α1-antitrypsin (A1AT; also known as α1-proteinase inhibitor and serpin A1). When it reaches the external face of the plasma membrane γ-tryptase can cleave and/or interact with proteins and peptides in the extracellular matrix. γ-tryptase is usually not released into the lumen. Although the γ-tryptase catalytic domains are 48% identical with those of the other mast cell tryptases, substrate specificity studies revealed that γ-tryptase and β1-tryptase are functionally distinct. γ-tryptases are transcribed in lung and several other tissues including the lower esophagus muscularis layer, intestines ("*intestinal tryptase*")*,* mucosa of the traverse colon and sigmoid colon, the small intestine Peyer's patches, in the MALT system (mucosa associated lymphoid tissue), as well as in the mast cell line HMC-1 which has been derived from a patient with mast cell leukemia. γ-tryptase is the sole membrane-anchored tryptase in humans and it has also been reported interacting with the neuronal calcium sensor (NCS) family of proteins, notably neurocalcin.

A polyclonal antibody recognizing human γ-tryptase was obtained by challenging chickens with a keyhole limpet hemocyanin-conjugated synthetic peptide (SEQ ID NO1: CRRDYPGPGGSILQP) corresponding to residues 192-206 of human prepro-γ-tryptase (Caughey et al.). Otherwise, the tryptases cannot be separated easily by their chemical properties. However, studies have shown that an administration of recombinant γ-tryptase (but not recombinant β1-tryptase) into the trachea of mice leads to airway hyperresponsiveness (AHR) and increased expression of interleukin-13. This effect is very specific because γ-tryptase can only induce AHR in normal mice but not in transgenic mice that lack signal transducer and activator of transcription (STAT) 6 or the α-chain of the cytokine receptor that recognizes both IL-4 and IL-13. Administration of small amounts of recombinant, mature human β1-tryptase (but not pro-β1-tryptase or mature human α-tryptase) into the trachea of MC-deficient W/Wv mice on the other hand confers protective immunity during *Klebsiella pneumonia* infection of the lung without inducing airway hyperresponsiveness (AHR). These data in their entirety indicate that γ-tryptase is an exocytosed neutral protease and specific surface mediator (Wong et al., Biochemical and Functional Characterization of Human Transmembrane Tryptase (TMT)/Tryptase-γ, JBC 2002, 277(44): 41906-41915). It is therefore surprising that the γ-tryptase level in stool can serve as a biomarker for IBS. The present disclosure therefore provides a clinical test for diagnosis of IBS when the fecal biomarkers for IBD and other GI diseases are low or negative. The γ-tryptase level in stool seems to correlate with the severity of the gastrointestinal conditions.

A predetermined reference value as defined herein refers to a standard or reference state of a function as a basis for comparison. A predetermined reference value indicative of a healthy status refers to the maximum value of a relevant parameter or substance concentration, which is not exceeded by an average human subject that is not suffering from IBS; and it is usually obtained by statistical analysis of a relevant population of comparable subjects. A predetermined reference value of γ-tryptase can be used as a limit concentration, above which, values are interpreted as indication of the occurrence of IBS. In contrast, lower values indicate the absence of IBS.

EP 0 859 792 B1, EP 2 445 517 B1, EP 3 004 892 B1 teach antibodies and systems for measuring tryptase in blood or serum for diagnosis and evaluation of an allergenic response, systemic mastocytosis or a danger of anaphylaxis. Even feces has been tested for total tryptase using the ImmunoCAP^{®} Total Tryptase assay of Phadia (Uppsala, Sweden) but the authors found no detectable total tryptase (<0.1 µg/L) in stool samples from 79 patients with IBS-D and 20/21 healthy controls (Tooth D et al, Characterization of fecal protease activity in irritable bowel syndrome with diarrhea: origin and effect of gut transit, Gut 2014, 63(5):753-60). This finding suggests that the above mentioned tryptase assay could only detect α- and/or β-tryptase which are structurally and functionally different to γ-tryptase. Moreover, a group of patients with various types of food sensitivity was likely tested which excludes a diagnosis of IBS-D.

IBS patients generally have disparate views about the pathological causes of their symptoms which typically include many types of food intolerance. Although IBS has a strong psychological etiology, the elicitation of the emotional causes is difficult. The disclosed method and test for γ-tryptase in stool are advantageous and helpful because from the patient's viewpoint directed to the organic symptoms (abdominal pain, discomfort relieved by defecation, change in stool form, passage of mucus, abdominal bloating as well as altered stool passage and/or frequency). The quantitation of γ-tryptase in stool further allows monitoring and a focus on positive coping measures, thus continuity in subsequent care. The new biomarker of IBS which can be translated into a clinical setting, is easy to interpret and gives way to a holistic diagnosis of GI diseases using fecal biomarkers. According to the present disclosure, the test sample can be a standard extract of stool or feces as used for many other fecal biomarkers.

### EXAMPLES

### EXAMPLE 1 - Enzyme-linked immunosorbent assay (ELISA) for γ-tryptase in feces.

The presence of γ-tryptase in feces from subjects suffering from IBS and healthy subjects for control was tested using standard techniques. 46 subjects (median age, 52 years; range, 22 - 60 years) were included in this study, namely healthy subjects (10), patients suffering from IBS-predominant constipated and mixed (12), allergic reactions (9) and IBS-predominant diarrhea (15). 15 mg stool samples were collected using an IDK^{®} Stool Sample Application System (Immundiagnostik AG, Bensheim, Art. No. K 6998SAS) and extracted with 1.5 mL IDK extract^{®} according to the manufacturer's instructions (Immundiagnostik AG, Bensheim, Germany). The γ-tryptase concentration was determined from the fecal extract (1:100) using a newly developed ELISA (detection limit 0.5 ng γ-tryptase / g feces).

In brief, a 96-well microtiter plate (Nunc maxisorp, VWR) was coated with a specific monoclonal rat antibody against γ-tryptase (3 µg anti-human γ-tryptase(TPSG1)-antibody/mL) which showed no cross-reaction with other tryptase forms for 2 h at 37 °C. After 3x rinses with PBS buffer (50 mM, pH 7.4) wells were prefilled with 50 µl Tris-buffer (1 M, pH 9.5). 100 µl extract (1:10) was acid-treated (100 mM acetic acid, 5 min, RT) and pipetted to the Tris buffer in the wells. The microtiter plate was incubated at 4 degrees Celsius overnight. The wells were washed 3x with PBS (50 mM). 65 µl acetic acid (100 mM) added to each well followed by an incubation at RT for 5 minutes. 50 µl acid-treated fraction were transferred onto an untreated plate containing 50 µl Tris buffer (1 M, pH 9.5) per well. The wells were washed 3X with PBS-Tween (2.5 % v/v, PBST) and after another 60 min incubation at RT 200 µl blocking buffer (5 % BSA, 5 % Casein in PBST) added for another 60 minutes incubation at RT. The wells were 3x rinsed with PBST, 10 µl biotinylated secondary anti-rat antibody (0.25 µg/ml) added to each well. After 60 min incubation on a rocker at RT and 3x washes with PBST, 100 µl streptavidin (0.5 µg/ml) conjugated with horseradish peroxidase (HRP) were added, followed by another 15 min rocking at RT and washes (3X) with PBST. Finally, 100 µl TMB substrate was added for color development. The microtiter plate was incubated for 5 min at RT in the dark and the reaction was stopped by adding 100 µl sulfuric acid (1 M) per well. The plates were analyzed by an ELISA reader (Dynex, MRX) at wavelengths of 450 and 630 nm. All analyses were performed in duplicate. γ-tryptase standards (quantification range 1-100 ng γ-tryptase / g feces) were run on each microtiter plate.

Results: The average γ-tryptase concentration in feces of healthy controls was 9.85 ng γ-tryptase /g feces (n=25) p<0.01). IBS-C and IBS-M patients had an increased γ-tryptase concentration in feces (15.62 ng γ-tryptase /g feces (n=24) p<0.01). For IBS-D patients, an average value of 35.71 ng γ-tryptase / g feces (n=27) p<0.01) was obtained. This demonstrates the feasibility of a rapid immunological test for γ-tryptase in feces. Furthermore, even the preliminary example showed that the fecal γ-tryptase concentration allowed a setting of threshold and cut-off-ranges for a differentiation between subjects suffering from IBS-C/ IBS-M and IBS-D.

### EXAMPLE 2 - Another enzyme-linked immunosorbent assay for fecal γ-tryptase.

Stool samples from the same patient population were processed as described in Example 1. The γ-tryptase concentration was determined by a new ELISA test. In brief, a 96-well microtiter plate (Nunc maxisorp, VWR) was coated for 2 h at 37 degrees Celsius with a monoclonal antibody (3 µg/mL per well) raised against amino acids 1-275 representing full-length mast cell tryptase (H-9) of human origin (Santa Cruz Biotechnology Inc., US; Ref. sc-271095) and wells were rinsed (3x) with PBS buffer (50 mM, pH 7.4) was added. 100 µl fecal extract (1:10) was acid-treated in 100 mM acetic acid at RT for 5 minutes to breakup protein complexes and pipetted into the wells containing 50 µl 1 M Tris buffer, pH 9.5. The microtiter plate was incubated at 4 degrees Celsius overnight. The wells were washed 3X with PBS (50 mM) and 65 µl of acetic acid (100 mM) added to each well. After 5 min rocking incubation at RT 50 µl of the acid-treated fractions were transferred onto an untreated plate containing 50 µl Tris buffer (1 M, pH 9.5) per well. After another 60 minutes incubation at RT, the wells were 3x washed with PBS-Tween (2.5 % v/v), and 200 µl blocking buffer (5 % BSA, 5 % Casein in PBST) added. After another 60 min incubation at RT and three washes of the wells with PBST, 1 µl monoclonal mouse antibody raised against purified lung mast cell tryptase (AA1) of human origin (Santa Cruz Biotechnology Inc., Ref. sc-59587) (0.2 µg/ml) was added to each well. After 60 min rocking at RT and 3X washing with PBST, 10 µl biotinylated rat anti-mouse antibody (0.25 µg/ml) was added and incubated for 1 h. Then, 100 µl streptavidin (0.5 µg/ml) conjugated with horseradish peroxidase (HRP) were added to each well, followed by another 15 min rocking at RT and washes (3X) with PBST, followed by an addition of 100 µl TMB substrate. For color development, the plate was incubated for 5 min at RT in the dark. The reaction was stopped by the addition of 100 µl sulphuric acid (1 M) per well. The plates were analyzed using an ELISA reader (Dynex, MRX) at wavelengths of 450 and 630 nm. All analyses were done in duplicates. γ-tryptase standards (quantification range 1-100 ng γ-tryptase / g feces) were run on each microtiter plate. Results: The average γ-tryptase concentration in feces from healthy controls was 9.71 ng γ-tryptase /g feces (n=25) p<0.01). IBS-C and IBS-M patients showed an increased γ-tryptase concentration (14.22 ng γ-tryptase / g feces (n=24) p<0.01). For IBS-D patients, an average value of 37.51 ng γ-tryptase / g feces (n=27) p<0.01) was obtained. A reliable immunological test with a novel marker of IBS in feces, without the requirement of spiking the samples with γ-tryptase, is thereby provided. Determination of fecal γ-tryptase concentration allows for a clear differentiation between subjects suffering from IBS-C/ IBS-M and IBS-D.

### EXAMPLE 3 - Stool sample extraction for immunoturbidimetry

Stool samples were extracted as follows. 15 mg stool was each diluted 1:100 in 1.5 ml IDK Extract^{®} extraction buffer (Cat. No. K 6967) at room temperature. Stool samples were collected and stored up to 48 h at 2-8°C. For long term periods (up to 12 months) storage at -20°C is recommended. Upon start of the turbidimetric assay, frozen samples were slowly thawed, preferably at 2-8°C. In some occasions, heterogeneous samples were homogenized mechanically. For sample collection, IDK Stool Sample Application System (SAS) (Cat. No. K 6998SAS) was used. The tip of the dipstick of SAS, which has notches that retain a fixed amount of raw material, was inserted into the stool sample. The dipstick was placed back into the tube with extraction buffer. When putting the stick back into the tube, excess material was stripped off. 15 mg stool sample remaining on the dipstick were then diluted in extraction buffer. The tubes were tightly closed and well shaken until no stool sample remained in the notches. 10 minutes were necessary to allow the sediment to settle. Making sure that the sediment was not dispersed again, the extracted sample was diluted 1:25 in sample buffer. For example, 40 µl extracted stool sample were added to 960 µl sample buffer. A final 1 :2500 dilution was obtained.

### EXAMPLE 4 - Production of immunoparticles against γ-tryptase

Latex particles from IKERLAT polymers, Lasarte-Oria, Spain (Ref. AJ17COOH) were used. The latex type was carboxylated polystyrene with particle sizes ranging from 90 to 250 nm. The latex particles had following parameters: surface charge density 53,0 µC/cm2; surface charge density 181,7 µEq/g pol.; solids content 10,0 %, stabilized with 0,05 % sodium azide. Immunoparticles were prepared by covalently attaching purified rat monoclonal antibodies against γ-tryptase to uniform carboxylated polystyrene particles. The reaction buffer was 3-(N-morpholino) propanesulfonic (MOPS) buffer pH = 7.2.

### EXAMPLE 5 - γ-Tryptase turbidimetric immunoassay

Samples extracted with either extraction buffer as described above were used for turbidimetric determination using the application Roche Hitachi 912 according to the manufacturer. 10 µL extracted sample were added to 200 µL test buffer and 50 µL storage buffer, comprising sodium citrate tribasic dehydrate, bovine serum albumin, Tween 20, sucrose, sodium azide. The immunoparticles were gently mixed with the extracted samples and incubated for 1-2 minutes at room temperature before running the automated analyzer. Measurements were performed in duplicates. The absorbance values were obtained reading at 570 nm wavelength. Commercially available purified γ-tryptase was diluted in a phosphate buffer pH=7.4 in a range from 1 to 100 ng γ-tryptase / mL for calibration of the turbidimetric assay. Stool samples with known amounts of γ-tryptase were used as control and test samples. Stool samples containing γ-tryptase were diluted 1:100 in reaction buffer, resulting in measurement ranges from 1 to 40 ng γ-tryptase in 1 g stool.

### EXAMPLE 6 - Question panel for identification of symptoms associated with IBS.

The questionnaire included questions directed to identifying the presence, severity, frequency, and/or duration of IBS-related symptoms such as abdominal pain, abdominal discomfort, constipation, diarrhea, bloating, and/or abdominal distension for confirmation of the diagnosis.

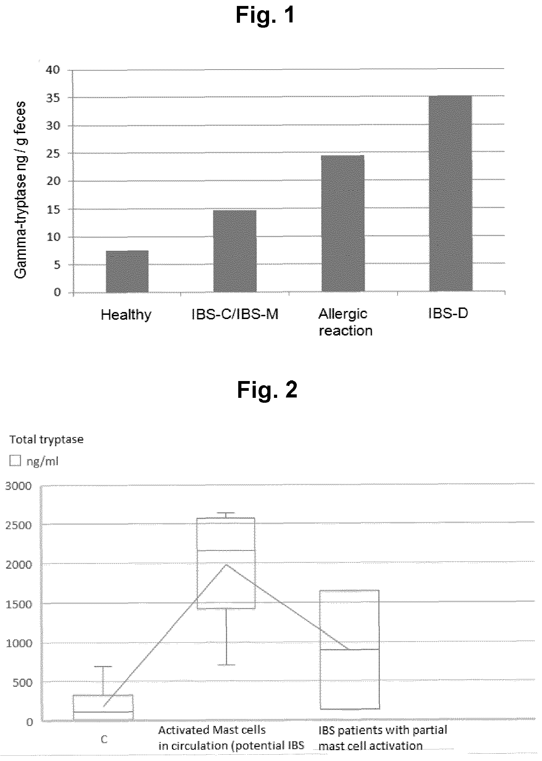

Figure 1 shows a comparison of average values of γ-tryptase concentration per gram feces from healthy subjects (10) and patients suffering from IBS-predominant constipated and mixed (12), allergic reactions (9) and IBS-predominant diarrhea (15) performed by ELISA using polyclonal total tryptase antibody as capture antibody and a specific monoclonal antibody as detection antibody. We have further found that γ-tryptase has diagnostic significance in discerning the occurrence of irritable bowel syndrome which had so far been carried out by an exclusion of other causes for the syndromes. Consequently, fecal γ-tryptase is a positive biomarker for diagnosis of irritable bowel syndrome (IBS) when the other biomarkers for IBD, leaky gut, inflammation and food allergies are low or negative.

### COMPARATIVE EXAMPLE 7 - Determination of total tryptase in feces

Total tryptase was extracted from random feces as described above and determined using a proprietary commercial ELISA for determination of tryptase in serum. The feces (without dietary limitation) from subjects (20) without gastrointestinal complaints and from subjects (8) either suffering from diagnosed IBS or a disease of immunological or allergic origin. The fecal samples were of arbitrary nature, say the subjects had not been subject to any specific dietary requirements. The median for tryptase was 136 ng/ml (average value 294 ng/mg) in subjects without GI symptoms and 1650 ng/g in patients with diagnosed IBS or immunological or allergic symptoms. Consequently, the concentration of tryptase in feces allowed no diagnosis of IBS but was indicative of irksome GI symptoms due to immunologic or allergic reactions. The normal value for total tryptase in the stool of healthy persons can be set at about 300 ng/ml.

While not wishing to be bound by theory, the literature suggests that the mucosa of patients with irritable bowel disease contains a high density of mast cells, likely in response to stimuli and signals from inflammatory (e.g. post-infectious) or allergic processes. Activated mast cells generally respond by a release of specific mediators, histamine and tryptase whereas activated intestinal mast cells release an intestinal γ-tryptase. The intestinal histamine is only detectable for a short period after stimulus, but the present inventors have found that the intestinal γ-tryptase is stable in feces and released from or with the membrane of mast cells for a much longer period so that the concentration of γ-tryptase in feces represents a diagnostic window for IBS. On the other hand, total tryptase is a biomarker for various allergic and immune reactions and therefore its concentration in feces is indicative for a plethora of allergic and immune diseases. Stress in general may also increase the amount of total tryptase in stool due to the neuro-immune crosstalk within the gut (Stakenborg N et al. in Intestinal neuro-immune interactions: focus on macrophages, mast cells and innate lymphoid cells, Neurobiology 2020, 62:68-75).

Retrospective studies have shown that allergy sufferers are at increased risk of IBS (Fang ZY et al, in Association between Allergic Diseases and Irritable Bowel Syndrome: A Retrospective Study in Int Arch Allergy Immunol 2018, 177:153-159). Preschool children with allergic rhinitis are at increased risk of IBS, and IBS patients are known to suffer more likely from allergic rhinitis. About 20% of the population worldwide suffer from allergies such as allergic rhinitis, asthma, urticaria, eczema, atopic dermatitis and various food allergies so that this primary condition may cause gastrointestinal problems independent from IBS while associated with increased risk for IBS.

Nickel is a known contact allergen and its concentration is increased in tomatoes, cocoa, beans, mushrooms, large-leaved vegetables, whole-meal flour, corn, onions, garlic, shellfish and nuts. Food may further be nickel contaminated if in an aluminum packaging. Nickel sensitivity can be detected in about 30 % of people living in Europe (Borghini R et al in New insights in IBS-like disorders: Pandora's box has been opened; a review. Gastroenterol Hepatol Bed Bench 2017, 10(2):79-89). They are also more likely of developing a chronic functional bowel disorder combined with an increased risk of IBS. In addition, many foods contain substances such as salicylates, bioactive amines, benzoate, and glutamate which may contribute to intestinal mast cell activation. It is known that avoidance of these substances often results in an improvement of irritable bowel symptoms. (De Giorgio R et al., in Sensitivity to wheat, gluten and FODMAPS in IBS: facts or fiction? Gut 2015, doi:10.1136/gutjnl-2015-309757). The association between IBS and numerous other diseases is independent and statistically significant (Koloski N et al. in Population based study: atopy and autoimmune diseases are associated with functional dyspepsia and irritable bowel syndrome, independent of psychological distress. Aliment Pharmacol Ther. 2019;1-10). Table I below summarizes the found associations between IBS and asthma, food allergy, psoriasis and rheumatoid arthritis.

**TABLE 1**

| *Assessment of various underlying diseases predisposing the development of IBS* | | | |
|---|---|---|---|
| Disease | Risk (Odds Ratio) | 95%- confidence interval | Significance P |
| Asthma | 1,55 | 1,20-1,99 | 0,001 |
| Food allergies | 1,85 | 1,31-2,61 | < 0,001 |
| Psoriasis | 2,03 | 1,38-3,02 | < 0,001 |
| Rheumatoid arthritis | 1,58 | 1,12-2,23 | 0,009 |

In our study, subjects suffering from one of the above underlying diseases (psoriasis, hay fever, food intolerance, rheumatoid arthritis) had conspicuously high intestinal total ryptase concentrations in stool. They can be assigned to the group of persons with gastrointestinal complaints which is not IBS ("potential IBS"). Three gastroenterology patients with unknown courses of disease and gastrointestinal complaints had increased intestinal total tryptase in stool. The results have also been schematically summarized in Fig. 2. The test therefore proved suitable for distinguishing IBS from gastrointestinal complaints of allergic or immunological origin, say patients with an increased risk of developing IBS. Thus, a treatment of the underlying disease as well as dietary and nutritional counselling may prevent or delay the onset of IBS and full development of gastrointestinal problems.

### Discussion and synopsis of results

Fig. 1 shows that patients with food allergy also have increased intestinal γ-tryptase in stool compared to healthy persons which is a sign for activated intestinal mast cells which can be associated to the development of IBS (Barbara et al. in Activated Mast Cells in Proximity to Colonic Nerves Correlate with Abdominal Pain and Irritable Bowel Syndrome. Gastroenterology2004, 693-702). Food allergies may also lead to a leaky inflammatory gut and can be diagnosed using suitable biomarkers in plasma and stool. The changes in the number and activation status of intestinal mast cells seems always associated with the symptoms of the irritable bowel syndrome (IBS) (Borghini et al, New insights in IBS-like disorders: Pandora's Box has been opened; a review. Gastroenterology and Hepatology From Bed to Bench 2017, 10(2):79-89). The present inventors have found that a measurement of γ-tryptase in stool is therefore a good biomarker for activated intestinal mast cells.

On the other hand, the serum α- and/or β-tryptases activate protease activated receptor type 2 (PAR2) which in turn increases the epithelial permeability while no reliable diagnostic cut-off ranges can be found for α- and/or β-tryptase in stool. Mast cells (MCs)1 can be generally held responsible for protecting the organism from foreign substances and pathogens. The mast cells release messenger substances (histamine, α- and/or β-tryptase) as mediators to activate various defense mechanisms and physiological changes (e.g. vasodilation, edema, smooth muscle contraction, and leukocyte recruitment). All human mast cells contain tryptase in the cell's granules. The respective 14 serine protease-like genes are present at the chromosome 16p13.3locus, including the gene that encodes γ-tryptase or transmembrane tryptase (Wong et al., Biochemical and Functional Characterization of Human Transmembrane Tryptase (TMT)/Tryptase-γ, JBC 2002, 277(44):41906-41915; Payne V, Kam PC. Mast cell tryptase: a review of its physiology and clinical significance, Anaesthesia 2004, 59(7):695-703). The α/β-tryptase level from serum mast cells is increased in immunologically mediated reactions following direct mast cell activation, e.g. anaphylaxis and allergic conditions, and their measurement in blood can be used to distinguish mast cell-dependent reactions from other systemic disturbances and clinical manifestations such as cardiogenic shock. Little is known however of the functions of membrane-bound intestinal γ-tryptase. Thus, it is surprising that its level in stool is distinguishably associated with the irritable bowel syndrome (IBS). The present application provides a method of determination of intestinal γ-tryptase and a cut-off value as a biomarker in stool which provides the practitioner and clinician a strong parameter for distinguishing IBS, IBD, hypochondriasis and various gastrointestinal disorders. The complexity of diagnosing IBS and IBD is simplified by performing a simultaneous measurement of γ-tryptase, total tryptase, calprotectin, hemoglobin, bile acids in feces. This can be done by using an immunoassay specific for γ-tryptase.

According to the present disclosure and our preliminary data, concentrations above > 30 ng γ-tryptase / g feces indicate irritable bowel syndrome, in particular IBS-C / IBS-M. Allergic reactions and food intolerances go in line with concentrations above > 300 ng total tryptase / g but also with increased levels of other plasma biomarkers. Concentrations above > 300 ng total tryptase / g feces with values of histamine 3.5 times higher than basal are found in cases of intestinal mastocytosis. Food allergies and IgE hypersensitivities are associated with increased levels of histamine and calprotectin in stool but also the patient's age is important (Zhu Q et al., Upregulation of calprotectin in mild IgE-mediated ovalbumin hypersensitivity, Oncotarget 2017, 8(23): 37342-37354; Kasirga E., The importance of stool tests in childhood, Turk Pediatri Ars 2019, 54(3): 141-148) whereas a study on the frequency of abnormal fecal biomarker levels in patients with irritable bowel syndrome was basically inconclusive (Goepp J et al, Frequency of Abnormal Fecal Biomarkers in Irritable Bowel Syndrome, - Global Advances in Health and Medicine 2014, 3(3):9-15). In summary, the determination of fecal γ-tryptase in addition to the other fecal biomarkers (calprotectin, pancreatic elastase, total tryptase, etc. ) provides a useful tool for a positive diagnosis of IBS which a person skilled in the art will also derive from the diagnostic scheme given below in Table 2.

**TABLE 2**

| *Fecal Biomarkers and Diagnostic Scheme* | | | | | | |
|---|---|---|---|---|---|---|
| Fecal biomarker & Gastrointestinal disorder | Pancreatic elastase -1 | Bile acid | Hemoglobin occult blood | calprotectin | Intestinal total trypases | γ-tryptase |
| Pancreatic disorders | +++ | - | - | - | - | - |
| Gall bladder / diarrhea | - | +++ | - | - | - | - |
| Colorectal cancer | - | - | +++ | + | - | - |
| IBD (MC und CU) | + | + | + | +++ | + | + |
| Food allergy | - | - | - | + | +++ | ++ |
| IBS | # | # | # | # | + | +++ |

| | | | | | | |
|---|---|---|---|---|---|---|
| +++ strongly abnormal result (diagnosis requires further examination and treatment); ++ elevated result (diagnosis requires further examination and treatment) + potential additional result, # rarely abnormal result (secondary diagnostic indication) - : negative | | | | | | |

A key to achieving relief for irritable bowel syndrome (IBS) is the understanding that IBS is a complex motility (motor) and sensory disorder which require a differential diagnosis in relation to other allergic and immunological diseases which may likewise give a cause to abdominal discomfort and gastrointestinal complaints. IBS may have physical and stress-related dimensions. The first line of treatment for IBS therefore includes general measures such as: establishing an effective patient-physician relationship, obtaining education about IBS, and inducing and implementing lifestyle changes, which may be associated with the symptoms. The term "lifestyle" refers to things patients have control over:- dietary or stress-related factors or foods that worsen symptoms. If the abdominal discomfort or pain occur after eating, smaller and more frequent meals may be recommended. Increased stress may result in the onset or worsening of IBS symptoms and associated non-bowel symptoms such as fatigue or low energy. Proper rest and exercise can help reduce stress levels and positively influence IBS. If lifestyle changes do not completely relieve IBS symptoms, a number of medications may be helpful: Antispasmodics [e.g., dicyclomine (Bentyl), hyoscyamine (Levsin)] have limited benefit for treating IBS but may relieve abdominal pain or discomfort in some persons, if the symptoms occur soon after eating. Anti-diarrheal agents [e.g., loperamide (Imodium), diphenoxylate (Lomotil)] can be effective in preventing and relieving symptoms of diarrhea but may not be as helpful for the pain. Laxatives can help treat symptoms of constipation but not necessarily the pain and should be used only under the supervision of a physician. Anti-anxiety medications can be helpful for some people with IBS, particularly those with psychological distress. Individuals with more mild-moderate symptoms will only require medications now and then. For example, an anti-diarrheal or antispasmodic may be taken by a person with diarrhea-predominant IBS before leaving home or eating a meal. Individuals with constipation may benefit from bulking agents (provided they relieve and don't worsen symptoms) or laxatives on occasion. There are also effective medications available that relieve the pain and improve the changes in bowel habit. These may need to be taken on a more long-term basis, such as low-dose antidepressant agents or the relatively newer medications. Above all and prior any medical treatment, a differential diagnosis of IBS in relation to allergic and immunological diseases that may also lead to abdominal discomfort is necessary and provided by the instant application.

## Claims

1. A method of diagnosis of irritable bowel syndrome (IBS) and its severity in a patient suffering from gastrointestinal disorders, comprising the steps of:-
preparing a defined extract of fecal proteins from a stool sample of a patient suffering from gastrointestinal disorders in a buffer system which allows a stable definition of a reference range;
determining the presence and concentration of γ-tryptase in said fecal protein extract using immunological methods;
comparing said measured concentration of γ-tryptase in said fecal protein extract to a predetermined reference value; and
assessment of said patient by assigning an increased likelihood of irritable bowel syndrome (IBS) when said measured concentration of γ-tryptase in said fecal protein extract is higher than a predetermined reference value, or by assigning a decreased likelihood of irritable bowel syndrome (IBS) when said measured concentration of γ-tryptase in said fecal protein extract is lower than said predetermined reference value.

2. The method of claim 1, comprising the use of antibodies raised against human γ-tryptase isolated from lung tissue.

3. The method of claim 1, comprising the use of monoclonal antibodies specifically binding human γ-tryptase.

4. The method of any claim 1 to 3, wherein the immunological methods comprise the steps of
i) providing an antibody raised against total mast cell tryptase which antibody is bound to a solid phase;
ii) contacting said antibody against total mast cell tryptase with said fecal protein extract and providing conditions for the formation of a first immune complex;
iii) providing a second antibody specific for human γ-tryptase and providing conditions to allow the formation of a sandwich immune complex; and
iv) determining the amount of sandwich immune complex formed to determine the amount of γ-tryptase in said fecal protein extract.

5. The method of any claim 1 to 4, wherein the immunological method is an enzyme-linked immunosorbent assay (ELISA).

6. The method of any claim 1 to 3, wherein the immunological method is an immunoturbidimetric or immunonephelometric assay.

7. The method of any claim 1 to 6, wherein predetermined reference value is determined in stool from healthy subjects and said reference value is 10 ng γ-tryptase per gram feces.

8. The method of any claim 1 to 6, further comparing said measured concentration of γ-tryptase to a predetermined reference value, wherein said predetermined reference value is 20 ng γ-tryptase per gram feces, which reference value is associated with an increased likelihood of irritable bowel syndrome subtypes IBS-Constipation / IBS-Mixed.

9. The method of any claim 1 to 6, further comparing said measured concentration of γ-tryptase to a predetermined reference value which predetermined reference value is 30 ng γ-tryptase / g feces and associated with an increased likelihood of suffering irritable bowel syndrome subtype IBS-Diarrhea.

10. The method of any claim 1 to 9, wherein said fecal protein extract is further examined for the presence and concentration of total tryptase, including α-, β- and γ-tryptase.

11. The method of any claim 1 to 10, wherein the fecal protein extract is further examined for the presence of one or more of the following fecal biomarkers: calprotectin, pancreatic elastase (fecal elastase-1), chymotrypsin, lactoferrin, hemoglobin, hemoglobin-haptoglobin-complex, anti-transglutaminase antibodies, anti-gliadin-antibodies, secretory IgA, α-1-trypsin, albumin, EDN, lysozyme, b-defensin, bile acids, fecal fat, acid steatocrit, fecal sugars (fructose, galactose, sucrose, lactose malabsorption or intolerance).

12. The method according to any one of the preceding claims, wherein the method further comprises assessing and identifying at least one symptom and/or condition in said subject suspected of suffering from IBS, wherein said at least one symptom is selected from older age, fever, weight loss, rectal bleeding, vomiting, lactose intolerance, drug-induced diarrhea, postcholecystectomy syndrome, laxative abuse, parasitic diseases, eosinophilic gastritis or enteritis, microscopic colitis, small-bowel bacterial overgrowth, celiac disease, early inflammatory bowel disease, abdominal pain, abdominal discomfort, constipation, diarrhea, bloating, abdominal distension,

13. Kit of parts for determining the type and severity of a gastrointestinal disorder in a subject suspected of suffering from irritable bowel syndrome or hypochondriasis, comprising
a) a device for transferring a defined amount of feces in a buffer system to prepare an extract of fecal proteins; and
b) reagents for determining the concentration of γ-tryptase in the said extract of fecal proteins,
comprising
- ) a reagent containing antibodies capable of binding total mast cell tryptase and forming a first complex; and
- a reagent containing antibodies raised against γ-tryptase isolated human lung tissue and specifically binding γ-tryptase; and
- reagents for quantification of antibody-γ-tryptase complex in a method as claimed in any claim 1 to 10.

14. Kit of parts for determining the type and severity of a gastrointestinal disorder in a subject suspected of suffering from irritable bowel syndrome or hypochondriasis, comprising:
a) a reagent containing antibodies capable of binding total mast cell tryptase and forming a first complex; and
b) a reagent containing antibodies raised against γ-tryptase isolated human lung tissue and specifically binding γ-tryptase; and
c) reagents for quantification of antibody-γ-tryptase complex in a method as claimed in any claim 1 to 10.

15. Kit as claimed in claim 13 or claim 14, comprising
a) a reagent containing antibodies capable of binding total mast cell tryptase and forming a first complex; and
b) a reagent containing monoclonal antibodies specifically binding γ-tryptase; and
c) reagents for quantification of antibody-γ-tryptase complex in a method as claimed in any claim 1 to 10.

## Patentansprüche

1. Verfahren zur Diagnose des Reizdarmsyndroms (IBS) und seines Schweregrades bei einem Patienten, der an gastrointestinalen Störungen leidet, umfassend die folgenden Schritte
Herstellung eines definierten Extrakts von fäkalen Proteinen aus einer Stuhlprobe eines Patienten, der an gastrointestinalen Störungen leidet, in einem Puffersystem, das eine stabile Definition eines Referenzbereichs gestattet;
Bestimmung des Vorhandenseins und der Konzentration von γ-Tryptase in dem fäkalen Proteinextrakt mithilfe immunologischer Methoden;
Vergleich der gemessenen Konzentration von γ-Tryptase in dem fäkalen Proteinextrakt mit einem vorbestimmten Referenzwert; und
Beurteilung des Patienten durch Zuweisung einer erhöhten Wahrscheinlichkeit eines Reizdarmsyndroms (IBS), wenn die gemessene Konzentration von γ-Tryptase in dem fäkalen Proteinextrakt höher als ein vorbestimmter Referenzwert ist, oder durch Zuweisung einer verringerten Wahrscheinlichkeit eines Reizdarmsyndroms (IBS), wenn die gemessene Konzentration von γ-Tryptase in dem fäkalen Proteinextrakt niedriger als der vorbestimmte Referenzwert ist.

2. Verfahren nach Anspruch 1, umfassend die Verwendung von Antikörpern, die gegen aus Lungengewebe isolierter menschlicher γ-Tryptase generiert wurden.

3. Verfahren nach Anspruch 1, das die Verwendung von monoklonalen Antikörpern umfasst, die spezifisch an menschliches γ-Tryptase binden.

4. Verfahren nach einem der Ansprüche 1 bis 3, wobei die immunologischen Verfahren die folgenden Schritte umfassen
i) Bereitstellen eines Antikörpers, der gegen die gesamte Mastzellen-Tryptase erzeugt wurde, wobei der Antikörper an eine feste Phase gebunden ist;
ii) Inkontaktbringen des Antikörpers gegen die gesamte Mastzellen-Tryptase mit dem Fäkalproteinextrakt und Schaffung von Bedingungen für die Bildung eines ersten Immunkomplexes;
iii) Bereitstellen eines zweiten Antikörpers, der spezifisch für menschliche γ-Tryptase ist, und Schaffen von Bedingungen, die die Bildung eines Sandwich-Immunkomplexes ermöglichen; und
iv) Bestimmung der Menge des gebildeten Sandwich-Immunkomplexes, um die Menge von γ-Tryptase in dem fäkalen Proteinextrakt zu bestimmen.

5. Verfahren nach einem der Ansprüche 1 bis 4, wobei das immunologische Verfahren ein Enzym-Immunoassay (ELISA) ist.

6. Verfahren nach einem der Ansprüche 1 bis 3, wobei das immunologische Verfahren ein immunoturbidimetrischer oder immunonephelometrischer Assay ist.

7. Verfahren nach einem der Ansprüche 1 bis 6, wobei ein vorbestimmter Referenzwert im Stuhl von gesunden Personen bestimmt wird und der Referenzwert 10 ng γ-Tryptase pro Gramm Kot beträgt.

8. Verfahren nach einem der Ansprüche 1 bis 6, wobei ferner die gemessene Konzentration von γ-Tryptase mit einem vorbestimmten Referenzwert verglichen wird, wobei der vorbestimmte Referenzwert 20 ng γ-Tryptase pro Gramm Stuhl ist, wobei der Referenzwert mit einer erhöhten Wahrscheinlichkeit von Reizdarmsyndrom-Subtypen IBS-Verstopfung / IBS-Mischung verbunden ist.

9. Verfahren nach einem der Ansprüche 1 bis 6, wobei ferner die gemessene Konzentration von γ-Tryptase mit einem vorbestimmten Referenzwert verglichen wird, wobei der vorbestimmte Referenzwert 30 ng γ-Tryptase / g Fäkalien beträgt und mit einer erhöhten Wahrscheinlichkeit verbunden ist, an einem Reizdarmsyndrom-Subtyp IBS-Diarrhoe zu leiden.

10. Verfahren nach einem der Ansprüche 1 bis 9, wobei der fäkale Proteinextrakt ferner auf das Vorhandensein und die Konzentration der gesamten Tryptase, einschließlich α -, β - und γ-Tryptase, untersucht wird.

11. Verfahren nach einem der Ansprüche 1 bis 10, wobei der fäkale Proteinextrakt ferner auf das Vorhandensein von einem oder mehreren der folgenden fäkalen Biomarker untersucht wird: Calprotectin, Pankreas-Elastase (fäkale Elastase-1), Chymotrypsin, Lactoferrin, Hämoglobin, Hämoglobin-Haptoglobin-Komplex, Anti-Transglutaminase-Antikörper, Anti-Gliadin-Antikörper, sekretorisches IgA, α -1-Trypsin, Albumin, EDN, Lysozym, β-Defensin, Gallensäuren, fäkales Fett, saurer Steatokrit, fäkale Zucker (Fructose, Galactose, Saccharose, Lactosemalabsorption oder -intoleranz).

12. Verfahren nach einem der vorangehenden Ansprüche, wobei das Verfahren ferner das Beurteilen und Identifizieren mindestens eines Symptoms und/oder Zustands in dem Unterobjekt, bei dem der Verdacht besteht, dass es an IBS leidet, umfasst, wobei das mindestens eine Symptom ausgewählt ist aus älterem Alter, Fieber, Gewichtsverlust, rektalen Blutungen, Erbrechen, Laktoseintoleranz, Medikamenteninduzierte Diarrhoe, Postcholezystektomie-Syndrom, Abführmittelmissbrauch, parasitäre Erkrankungen, eosinophile Gastritis oder Enteritis, mikroskopische Kolitis, bakterielle Dünndarmüberwucherung, Zöliakie, frühe entzündliche Darmerkrankung, Bauchschmerzen, abdominelles Unbehagen, Verstopfung, Durchfall, Blähungen, abdominelle Distension,

13. Teilesatz zur Bestimmung der Art und des Schweregrades einer gastrointestinalen Störung bei einer Person, die im Verdacht steht, an einem Reizdarmsyndrom oder einer Hypochondrie zu leiden, bestehend aus
a) eine Vorrichtung zum Überführen einer definierten Menge von Fäkalien in ein Puffersystem, um einen Extrakt von Fäkalienproteinen vorzubereiten; und
b) Reagenzien zur Bestimmung der Konzentration von γ-Tryptase in dem genannten Extrakt aus fäkalen Proteinen, umfassend
- ein Reagenz, das Antikörper enthält, die in der Lage sind, die gesamte Mastzellen-Tryptase zu binden und einen ersten Komplex zu bilden; und
- ein Reagenz, das Antikörper enthält, die gegen γ-Tryptase isoliertes menschliches Lungengewebe gezüchtet wurden und spezifisch γ-Tryptase binden; und
- Reagenzien zur Quantifizierung des Antikörper- γ-Tryptase-Komplexes in einem Verfahren nach einem der Ansprüche 1 bis 10.

14. Kit zur Bestimmung der Art und des Schweregrades einer gastrointestinalen Störung bei einer Person, die im Verdacht steht, an einem Reizdarmsyndrom oder einer Hypochondrie zu leiden, umfassend
a) ein Reagenz, das Antikörper enthält, die in der Lage sind, die gesamte Mastzellen-Tryptase zu binden und einen ersten Komplex zu bilden; und
b) ein Reagenz, das monoklonale Antikörper enthält, die spezifisch γ-Tryptase binden; und
c) Reagenzien zur Quantifizierung des Antikörper-γ-Tryptase-Komplexes in einem Verfahren nach einem der Ansprüche 1 bis 10.

15. Kit nach Anspruch 13 oder Anspruch 14, umfassend
a) ein Reagenz, das Antikörper enthält, die in der Lage sind, die gesamte Mastzellen-Tryptase zu binden und einen ersten Komplex zu bilden; und
b) ein Reagenz, das monoklonale Antikörper enthält, die spezifisch γ-Tryptase binden; und
c) Reagenzien zur Quantifizierung des Antikörper-γ-Tryptase-Komplexes in einem Verfahren nach einem der Ansprüche 1 bis 10.

## Revendications

1. Méthode de diagnostic du syndrome du côlon irritable (SCI) et de sa gravité chez un patient souffrant de troubles gastro-intestinaux, comprenant les étapes suivantes:-
préparer un extrait défini de protéines fécales à partir d'un échantillon de selles d'un patient souffrant de troubles gastro-intestinaux dans un système tampon qui permet une définition stable d'une gamme de référence ;
déterminer la présence et la concentration de γ-tryptase dans ledit extrait de protéines fécales à l'aide de méthodes immunologiques ;
comparer ladite concentration mesurée de γ-tryptase dans ledit extrait de protéines fécales à une valeur de référence prédéterminée ; et
l'évaluation dudit patient par l'attribution d'une probabilité accrue de syndrome du côlon irritable (SCI) lorsque ladite concentration mesurée de γ-tryptase dans ledit extrait de protéine fécale est supérieure à une valeur de référence prédéterminée, ou par l'attribution d'une probabilité réduite de syndrome du côlon irritable (SCI) lorsque ladite concentration mesurée de γ-tryptase dans ledit extrait de protéine fécale est inférieure à ladite valeur de référence prédéterminée.

2. La méthode de la revendication 1, comprenant l'utilisation d'anticorps dirigés contre la γ-tryptase humaine isolée du tissu pulmonaire.

3. La méthode de la revendication 1, comprenant l'utilisation d'anticorps monoclonaux liant spécifiquement la γ-tryptase humaine.

4. Méthode selon l'une des revendications 1 à 3, dans laquelle les méthodes immunologiques comprennent les étapes suivantes
i) fournir un anticorps dirigé contre la tryptase totale des mastocytes, cet anticorps étant lié à une phase solide ;
ii) mise en contact dudit anticorps contre la tryptase mastocytaire totale avec ledit extrait de protéines fécales et mise en place des conditions nécessaires à la formation d'un premier complexe immun ;
iii) fournir un second anticorps spécifique de la γ-tryptase humaine et réunir les conditions permettant la formation d'un complexe immun en sandwich ; et
iv) déterminer la quantité de complexe immun en sandwich formé pour déterminer la quantité de γ-tryptase dans ledit extrait de protéines fécales.

5. La méthode de l'une des revendications 1 à 4, dans laquelle la méthode immunologique est un test immun-enzymatique (ELISA).

6. Méthode selon l'une des revendications 1 à 3, dans laquelle la méthode immunologique est un test immun-turbidimétrique ou immun-néphélé-métrique.

7. Méthode selon l'une des revendications 1 à 6, dans laquelle la valeur de référence prédéterminée est déterminée dans les selles de sujets sains et ladite valeur de référence est de 10 ng γ-tryptase par gramme de fèces.

8. La méthode de l'une des revendications 1 à 6, en comparant en outre ladite concentration mesurée de γ-tryptase à une valeur de référence prédéterminée, dans laquelle ladite valeur de référence prédéterminée est de 20 ng de γ-tryptase par gramme de fèces, cette valeur de référence étant associée à une probabilité accrue de syndrome du côlon irritable de sous-types IBS-Constipation / IBS-Mixte.

9. La méthode de l'une des revendications 1 à 6, en comparant en outre ladite concentration mesurée de γ-tryptase à une valeur de référence prédéterminée, laquelle valeur de référence prédéterminée est de 30 ng γ-tryptase / g de fèces et est associée à une probabilité accrue de souffrir du sous-type IBS-Diarrhée du syndrome de l'intestin irritable.

10. Méthode selon l'une des revendications 1 à 9, dans laquelle l'extrait de protéines fécales est examiné pour déterminer la présence et la concentration de tryptase totale, y compris la α-, la β- et la γ-tryptase.

11. La méthode de l'une des revendications 1 à 10, dans laquelle l'extrait protéique fécal est en outre examiné pour la présence d'un ou plusieurs des biomarqueurs fécaux suivants : calprotectin, élastase pancréatique (élastase fécale-1), chymotrypsine, lactoferrine, hémoglobine, complexe hémoglobine-haptoglobine, anticorps anti-transglutaminase, anticorps anti-gliadine, IgA sécrétoire, ?-1-trypsine, albumine, EDN, lysozyme, b-défensine, acides biliaires, graisses fécales, steatocrit, acide, sucres fécaux (fructose, galactose, saccharose, malabsorption ou intolérance au lactose).

12. Méthode selon l'une quelconque des revendications précédentes, dans laquelle la méthode comprend en outre l'évaluation et l'identification d'au moins un symptôme et/ou d'une condition chez le sujet suspecté de souffrir du SII, dans laquelle ledit au moins un symptôme est choisi parmi l'âge avancé, la fièvre, la perte de poids, les saignements rectaux, les vomissements, l'intolérance au lactose, diarrhée médicamenteuse, syndrome post-cholécystectomie, abus de laxatifs, maladies parasitaires, gastrite ou entérite éosinophile, colite microscopique, prolifération bactérienne dans l'intestin grêle, maladie coeliaque, maladie inflammatoire précoce de l'intestin, douleur abdominale, gêne abdominale, constipation, diarrhée, ballonnement, distension abdominale,

13. Kit de pièces permettant de déterminer le type et la gravité d'un trouble gastro-intestinal chez un sujet suspecté de souffrir du syndrome du côlon irritable ou d'hypocondrie, comprenant
a) un dispositif pour transférer une quantité définie de matières fécales dans un système tampon afin de préparer un extrait de protéines fécales ; et
b) des réactifs pour déterminer la concentration de γ-tryptase dans ledit extrait de protéines fécales, comprenant
- un réactif contenant des anticorps capables de se lier à la tryptase totale des mastocytes et de former un premier complexe ; et
- un réactif contenant des anticorps dirigés contre la γ-tryptase isolée du tissu pulmonaire humain et liant spécifiquement la γ-tryptase ; et
- des réactifs pour la quantification du complexe anticorps-γ-tryptase dans une méthode selon l'une des revendications 1 à 10.

14. Kit permettant de déterminer le type et la gravité d'un trouble gastro-intestinal chez un sujet suspecté de souffrir du syndrome du côlon irritable ou d'hypocondrie, comprenant
a) un réactif contenant des anticorps capables de se lier à la tryptase totale des mastocytes et de former un premier complexe ; et
b) un réactif contenant des anticorps monoclonaux liant spécifiquement la γ-tryptase ; et
c) des réactifs pour la quantification du complexe anticorps-γ-tryptase dans une méthode selon l'une des revendications 1 à 10.

15. Kit selon la revendication 13 ou la revendication 14, comprenant
a) un réactif contenant des anticorps capables de se lier à la tryptase totale des mastocytes et de former un premier complexe ; et
b) un réactif contenant des anticorps monoclonaux liant spécifiquement la γ-tryptase ; et
c) des réactifs pour la quantification du complexe anticorps-γ-tryptase dans une méthode selon l'une des revendications 1 à 10.
